# EUROPÄISCHE PATENTSCHRIFT

(11) **EP 2 382 916 B1**
(45) Veröffentlichungstag und Bekanntmachung des Hinweises auf die Patenterteilung: **27.06.2012**
(21) Anmeldenummer: 10004502.0
(22) Anmeldetag: 28.04.2010
(51) Int. Cl.: A61B 5/00

(54) **Vorrichtung und Verfahren zur Bestimmung des Fettgehaltes des menschlichen Körpers**
Method and device for determining the fat content of the human body
Dispositif et procédé de détermination de la teneur en graisses du corps humain

(43) Veröffentlichungstag der Anmeldung: 02.11.2011
(73) Patentinhaber: DERMALOG Identification Systems GmbH, 20148 Hamburg (DE)
(72) Erfinder: Lehmann, Eckhart, 22587 Hamburg (DE); Mull, Günther, 22085 Hamburg (DE); Hengfoss, Clarissa, 22305 Hamburg (DE); Kulcke, Axel, Dr., 8382 Weiselbaum (AT)
(74) Vertreter: UEXKÜLL & STOLBERG

(56) Entgegenhaltungen:
- EP-A1- 2 382 916
- US-A- 4 928 014
- US-B1- 6 285 904
- WEIJUN SONG ET AL: "The System of Portable Fat Detector With Dual-Wavelength Near-Infrared Light" BIOINFORMATICS AND BIOMEDICAL ENGINEERING , 2009. ICBBE 2009. 3RD INTERNATIONAL CONFERENCE ON, IEEE, PISCATAWAY, NJ, USA, 11. Juni 2009 (2009-06-11), Seiten 1-4, XP031489465 ISBN: 978-1-4244-2901-1

## Beschreibung

Die vorliegende Erfindung betrifft eine Vorrichtung zur Bestimmung des Fettgehaltes des menschlichen Körpers mit einer Lichtquelle zur Einstrahlung von Licht, das einen Anteil im NIR-Spektralbereich einschließt, in einen Körperteil, einer Lichtsensoreinrichtung zur Aufnahme von aus dem Körperteil entfernt von dem Einstrahlungsort auf derselben Seite des Körperteils wieder austretendem Licht, einer Spektrometereinrichtung zur differenzierten Erfassung von wenigstens zwei Spektralbereichen des wieder austretenden Lichts und mit einer Datenverarbeitungseinheit, die Messsignale der Lichtsensoreinrichtung aufnimmt und zur Bestimmung des Fettgehaltes des Körperteils verarbeitet.

Die einfache und zuverlässige Bestimmung des Anteils von Fett im Körper einer Person ist heutzutage eine sehr wichtige Aufgabe, da durch einen zu hohen oder zu niedrigen Fettanteil im Körper direkt oder indirekt verursachte Krankheiten auftreten können. Dabei ist die Fettleibigkeit (Adipositas) bereits als Krankheit definiert. Nach WHO-Richtlinien liegt ab einem Körpermasseindex (Body Mass Index) von 30 kg/m² Adipositas vor. Dadurch erhalten Messungen zur Bestimmung des Körperfettanteils neben der Anwendung im Fitness- und Sportbereich auch immer mehr Bedeutung im medizinischen Bereich. Auf der einen Seite führt ein zu hoher Fettanteil im Körper nicht nur zu orthopädischen Problemen, sondern erhöht auch substantiell die Gefährdung für Herzkrankheiten, Diabetes Mellitus, Schlaganfälle oder sogar für Krebs. Ein zu niedriger Fettanteil ist auf der anderen Seite ein Anzeichen für Mangelerscheinungen und kann auch zu schwerwiegenden gesundheitlichen Schädigungen führen.

Aus medizinischer Sicht betrachtet ist das Fettgewebe eine Spezialform des retikulären Bindegewebes. Das Fettgewebe macht rund 10-20% des Körpergewichtes eines normalgewichtigen Mannes und 15-25% des Körpergewichtes einer normalgewichtigen Frau aus. Das retikuläre Bindegewebe ist ein dreidimensionaler, schwammartiger Verband von Retikulinzellen und retikulären Fasern, in dessen Lücken die Fettzellen eingelagert werden. Fettzellen sind Zellen, in denen ein Fetttropfen von Zytoplasma umhüllt wurde. Chemisch gesehen sind Fetttropfen ein Gemisch aus Glyzerinester (Neutralfett), Öl-, Palmitin-, Stearinsäuren und Farbstoffen (Lipochrome). Fett liegt im lebenden Organismus meist im flüssigen Zustand vor. Das Fettgewebe wird unterteilt in Baufett (viszerales Fett) und Speicherfett (subkutanes Fett). Das Baufett dient als Polstermaterial, zur Erhaltung der Organlage und als Gewebeersatz. Das Speicherfett dient als Energiespeicher, elastisches Polster bei mechanischen Einwirkungen und thermischer Isolator zur Erhaltung der Körpertemperatur. Speicherfett befindet sich vor allem im Unterhautbindegewebe (subkutan) und in der Bauchhöhle. Das Baufett ist in dem Gewebe von Muskeln und Organen eingelagert. Es ist bekannt, dass eine erhöhte Menge an subkutanem Speicherfett für sich alleine betrachtet medizinisch unbedenklich ist, da es nicht direkt Krankheiten verursacht. Hier sind eher die ästhetischen Gesichtspunkte wichtig. Es wäre also wünschenswert, wenn bei der Fettanteilbestimmung zwischen viszeralem und subkutanem Fett unterschieden werden könnte.

Medizinisch relevant sind die Baufetteinlagerungen im Organgewebe. Diese führen im Allgemeinen zu den bereits genannten gesundheitlichen Problemen. Die Konzentrationen von viszeralem Fett korrelieren zwar mit den Anteilen von subkutanem Fett, sollten aber bei einer Analyse voneinander unterschieden und einzeln erfasst werden. Die vorliegende Erfindung zielt auch darauf ab, die unterschiedlichen Fettanteile gesondert erfassbar zu machen. Dazu soll nach der vorliegenden Erfindung eine tiefenabhängige Bestimmung des Fettanteils erfolgen, d.h. des Fettanteils als Funktion der Tiefe unter Haut.

Der Fettauf- und -abbau wird im Wesentlichen durch die Art und den Umfang der Nahrungsaufnahme sowie der körperlichen Aktivität bestimmt. Daher kommen Geräte zur Fettanalyse auch hauptsächlich in medizinischen Einrichtungen, in Fitness-Centern und bei Sportmannschaften zum Einsatz. Dabei dienen die Geräte zum einen der Bestimmung des aktuellen Gesamtfettanteils im Körper, zum anderen erlauben sie die Veränderungen während der Diät- oder Fitnessprogramme mit zu überwachen.

Es gibt unterschiedliche wissenschaftliche Methoden zur Gesamtfettbestimmung im menschlichen Körper. Darunter sind sehr genaue Verfahren, die aber aufgrund des Aufwands oder der Kosten nur in wissenschaftlichen Studien angewendet werden. Zu diesen Verfahren gehören das Unterwasserwiegen (Hydrodensitometrie), die DEXA (Dual-Energy X-Ray Absorptiometry), die Deuteriumdioxid-Verdünnungsmethode (D₂O Dilution) und die Kernspintomographie (MRT).

Weiterhin gibt es sehr einfache Verfahren zur Gesamtfettbestimmung über die Berechnung des Body Mass Indexes BMI = m/l² mit m = Körpermasse in kg und l = Körpergröße in m. Aus neueren Studien ist allerdings bekannt, dass bei alleiniger Betrachtung des BMI Fehlklassifizierungen erfolgen können. Das liegt daran, dass der Körperbau und der Trainingszustand der Muskeln hierbei nicht beachtet werden. Es wird allein aus Größe und Gewicht auf den "Körperfettanteil" geschlossen. Zum einen gibt es dadurch Überschätzungen des Fettanteils bei Personen mit gut trainiertem Muskelgewebe, da Muskelmasse schwerer als Fettgewebe ist. Zum anderen gibt es Personen, die einen BMI im Normalbereich haben, aber trotzdem einen als medizinisch kritisch zu bewertenden Fettanteil besitzen.

Daher kommen in neuerer Zeit weitere Methoden zur Körperfettbestimmung über die Bioimpedanzmessung, die NIR-Spektroskopie und die Ultraschallbestimmung hinzu. Alle eben genannten Methoden sind relativ kostengünstig und ermöglichen genaue Messungen mit einem vertretbaren Zeitaufwand.

Die vorliegende Erfindung ist auf die Bestimmung des Fettanteils unter Anwendung von NIR-Spektroskopie gerichtet, deren Gültigkeit bereits in vielen wissenschaftlichen Arbeiten nachgewiesen wurde.

Eine der ersten allgemein akzeptierten Arbeiten hierzu war die Studie von Conway et al. "A New Approach for the Estimation of Body Composition: Infrared Interactance", American Journal of Clinical Nutrition 40, December 1984, Seiten 1123 - 1130. In dieser Studie wurde über ein Lichtleiterfaserbündel Licht aus einer spektral durchstimmbaren Lichtquelle in einen Körperteil eingestrahlt und Licht, das durch mehrfache diffuse Streuung im Gewebe an einer anderen Stelle wieder austrat, über ein Lichtleiterfaserbündel zu einem Detektor geleitet. Die Lichtquelle wurde dann so gesteuert, dass in der Wellenlänge Durchläufe von 700 bis 1.100 nm durchgeführt wurden, wodurch die Spektren für verschiedene Körperteile und verschiedene Testpersonen aufgenommen wurden.

Die Bestimmung des Fettanteils kann an unterschiedlichen Körr perteilen durchgeführt werden. Sehr häufig wird als Referenzstelle die Mitte des Bizepses der bevorzugten Handseite ausgewählt. Messungen werden aber auch auf dem Trizeps oder an den Hautfalten an der Taille (suprailiakal) durchgeführt. Bei den Messungen am Bizeps wurde in unterschiedlichen Veröffentlichungen nachgewiesen, dass die ermittelten Ergebnisse an dieser Körperstelle mittels eines Korrelationsfaktors auf den prozentualen Körperfettanteil insgesamt übertragen werden können.

Für die Gerätetechnik und Messungen im Feld wurden einfachere Geräte vorgestellt, welche im Allgemeinen mit LEDs als spektral eingeschränkte Beleuchtungsquellen und mit einem einfachen Detektor arbeiten. In anderen Fällen wurden breitbandige Beleuchtungseinheiten in Kombination mit Detektoren, die durch optische Filter spektral eingeschränkt wurden, eingesetzt. Eine Vorrichtung der zuerst genannten Art ist zum Beispiel in U.S. Patent 4,928,014 beschrieben, auf die der Oberbegriff von Patentanspruch 1 zurückgeht.

Die in diesem U.S. Patent 4,928,014 beschriebene Vorrichtung weist eine Lichtquelle in Form von im NIR-Bereich strahlenden Leuchtdioden auf, die in gleichem Abstand einander gegenüberliegend zu einem mittig angeordneten Lichtsensor angeordnet sind. Die Spektrometereinrichtungen sind dadurch realisiert, dass die eine Leuchtdiode ihr Emissionsmaximum in einem ersten Wellenlängenbereich im NIR-Spektrum hat, und die andere Leuchtdiode ihr Emissionsmaximum in einem von dem ersten Spektralbereich verschiedenen zweiten Spektralbereich hat. Die Steuerung der Leuchtdioden erfolgt so, dass die Leuchtdioden separat und aufeinanderfolgend betätigt werden, so dass aufeinanderfolgende Messungen in zwei Spektralbereichen erhalten werden. Durch diese Anordnung ist eine Spektrometereinrichtung mit niedrigster Auflösung, nämlich zwei Spektralbereichen, gegeben, wobei die eine Leuchtdiode ihr Emissionsmaximum in einem für Wasser charakteristischen Spektralbereich und die andere in einem für Fett charakteristischen Spektralbereich haben kann. Die spektrale Sensitivität und Messgenauigkeit ist aber gering.

Eine andere Vorrichtung zur Bestimmung des Fettanteils ist in U.S. 2004/0092803 beschrieben. Die Vorrichtung verwendet eine breitbandige, den NIR-Bereich abdeckende Lichtquelle und ein FT-NIR-Spektrometer (Fourier Transform Near Infrared), wobei Licht über ein Lichtleiterfaserbündel auf das Ohrläppchen eingestrahlt und reflektiertes Licht über das Lichtleiterfaserbündel einem Detektor zugeführt wird. Daraus kann das Spektrum des reflektierten Lichts abgeleitet werden. Diese Messvorrichtung hat aber viele Nachteile. Sie ist zum einen apparativ sehr aufwändig, da FT-NIR-Spektrometer apparativ kompliziert und teuer sind. Zum anderen dauert eine Messung relativ lange, wobei beispielhaft fünf Integrationen in etwa einer Minute in der Druckschrift genannt werden. Ferner ist für diese Vorrichtung zur Messung ein Spektralbereich um 1,25 µm vorgesehen, da dieser Spektralbereich für FT-NIR-Spektrometer und daher für die beschriebene Vorrichtung geeignet ist; andererseits ist dieser Spektralbereich aber für die Messung des Fettanteils in Körperteilen des menschlichen Körpers nachteilig. Im menschlichen Gewebe ist eine hohe Transmission nur im Spektralbereich von etwa 700 nm bis 1.000 nm gegeben. Bei der Verwendung eines darüberliegenden Spektralbereichs von über 1 µm nehmen die effektiven Eindringtiefen stark ab, da das Wasser im Gewebe das Licht nahezu vollständig absorbiert. Das hat zur Folge, dass bei den Messungen stärkere Abweichungen auftreten, die durch Absorption und Reflexion an der obersten Hautschicht entstehen, d.h. die beschriebene Vorrichtung hat nur eine äußerst niedrige Sensitivität für die Gegebenheiten im Gewebe unterhalb der Haut. Ferner ist für diese beschriebene Vorrichtung vorgesehen, dass die Messung in Reflexion durchgeführt wird. Es ist aber aus der medizinischen Literatur bekannt, dass eine quantitative Bestimmung von Gewebeanteilen im Allgemeinen nur im sogenannten Transflexionsverfahren exakt durchführbar ist. Ein Transflexionsverfahren ist dadurch gekennzeichnet, dass Licht aus einer Lichtquelle ohne einen großen Brechungsindexunterschied in das Gewebe eingekoppelt wird, wonach es in dem Gewebe mehrfach diffus gestreut wird und so bis zu einer definierten Austrittsstelle wandert. Beim Transflexionsverfahren wird dann das an der Austrittsstelle ausgetretene Licht spektral analysiert.

Weiterhin wurden in U.S. 2004/0092803 bei der Messung am Ohrläppchen nicht die wechselnden Umgebungslichtverhältnisse berücksichtigt. Trotz höherer Absorptionseigenschaften des Gewebes im oben genannten Spektralbereich können geringe Mengen von Fremdlicht durch das dünne Ohrläppchen hindurchtreten und die Messung deutlich verfälschen. Somit ist die vorgeschlagene Vorrichtung auch in diese Punkt als kritisch zu betrachten.

Aus WO2008/029978 A1 ist eine Messvorrichtung zur Messung der Dicke der subkutanen Fettschicht bekannt. Die Vorrichtung weist eine breitbandige Lichtquelle zum Beispiel mit einem Spektralbereich von 400 nm bis 2.500 nm auf, mit der Licht in den zu messenden Körperteil einstrahlbar ist. In unterschiedlicher Entfernung von dem Einstrahlungspunkt der Lichtquelle befinden sich zwei Lichtsensoren, die jeweils mit einem Filter versehen sind, die jeweils nur Licht im NIR-Bereich von 1.000 nm bis 1.400 nm passieren lassen. Eine spektrale Aufteilung oder Auflösung wie in der gattungsgemäßen Vorrichtung ist damit nicht möglich. Zur Auswertung der detektierten Signale der Lichtsensoren wird lediglich erläutert, dass die Amplitude der Messsignale mit der Amplitude eines gespeicherten Referenzsignals verglichen und daraus eine Dicke der subkutanen Fettschicht abgeleitet wird. Bei dem angegebenen Spektralbereich von 1.000 nm bis 1.400 nm resultieren die Messsignale immer aus Überlagerungen von Wasser, Proteinen, Fett und anderen Gewebebestandteilen, so dass die Sensitivität und Messgenauigkeit zur Bestimmung des Fettanteils sehr begrenzt ist.

Den vorhergehend beschriebenen und anderen im Stand der Technik bekannten Verfahren der Fettgehaltserfassung mittels NIR-Spektroskopie ist gemeinsam, dass sie keinerlei Sensitivität für die Erfassung unterschiedlich tiefer Gewebeschichten aufweisen, d.h. sie lassen keinerlei Rückschlüsse auf die Verteilung des Fettanteils als Funktion der Tiefe unter der Hautoberfläche zu. Somit ist auch keine Unterscheidung in subkutanes oder viszerales Fett möglich, welches aber eine der wichtigen Informationen für die medizinische Beurteilung ist.

Es ist Aufgabe der vorliegenden Erfindung, eine Vorrichtung und ein entsprechendes Verfahren anzugeben, die einerseits einen einfachen apparativen Aufbau erlauben, andererseits eine sensitive und exakte Erfassung des Fettanteils in Körperteilen eines Menschen ermöglichen und darüber hinaus sensitiv für den Fettgehalt als Funktion der Tiefe unter der Hautoberfläche sind und eine Tiefendiskriminierung der Fettgehaltsbestimmung erlauben.

Diese Aufgabe wird gelöst durch eine Vorrichtung mit den kennzeichnenden Merkmalen des Patentanspruchs 1 in Verbindung mit dessen Oberbegriff sowie durch ein Verfahren mit den kennzeichnenden Merkmalen des Anspruchs 9 in Verbindung mit dessen Oberbegriff. Vorteilhafte Ausführungsformen der Erfindung sind in den Unteransprüchen angegeben.

In der erfindungsgemäßen Vorrichtung ist die Spektrometereinrichtung als ein zweidimensionales Spektrometer in folgender Weise aufgebaut: Es hat eine schlitz- oder spaltförmige Blende zur Ausblendung eines schlitzförmigen Bereichs von aus dem untersuchten Körperteil entfernt von dem Einstrahlungsort wieder austretenden Lichts; dabei wird auf der gleichen Seite des Körperteils wieder austretendes Licht erfasst, d.h. es wird keine Durchstrahlungsanordnung realisiert. Die Blende selektiert einen streifenförmigen Bereich des austretenden Lichts, der entfernt vom Einkopplungsort der Lichtquelle liegt und entlang dessen daher die Entfernung zum Einkopplungsort variiert. Vorzugsweise kann die Blende so liegen, dass der Einkopplungsort der Lichtquelle auf der Verlängerung der Längsachse des streifenförmigen Bereichs diesem benachbart liegt, so dass die Entfernung zum Einkopplungsort von dem diesem benachbarten Ende ausgehend entlang des schlitzförmigen Bereichs linear zunimmt. Ferner ist in dem Spektrometer ein Gitter vorgesehen, das so aufgebaut und angeordnet ist, dass die durch die Beugung am Gitter bewirkte dispersive Auffächerung des durch die Blende hindurchtretenden Lichts in einer von der Längsrichtung der Blende verschiedenen, vorzugsweise senkrecht dazu liegenden, Richtung erfolgt. Von dem Gitter gebeugtes Licht wird auf einen Kamerasensor geworfen, der so aufgebaut und angeordnet ist, dass er ein Beugungsbild des Gitters mit der Längsrichtung der Blende in einer ersten Richtung und der dispersiven spektralen Auffächerung des Lichts in einer von der ersten verschiedenen zweiten, vorzugsweise dazu senkrechten Richtung auf seiner Kamerasensorfläche aufnimmt. Der Kamerasensor ist mit der Datenverarbeitungseinheit verbunden, die weiter dazu vorbereitet ist, die von dem Kamerasensor empfangenen Signale als eine Vielzahl von Spektren entsprechend einer Vielzahl von Bildelementen entlang der Längsrichtung der Blende aufzunehmen und die Vielzahl von Spektren in Zuordnung zu den Orten entlang der Längsrichtung der Blende und damit der Entfernung vom Einstrahlungsort multivariaten statistischen Analysen zu unterziehen, um den Anteil von Fett aus den Spektren als Funktion der Entfernung vom Einstrahlungsort und damit als Funktion der Tiefe der erfassten Schichten unter der Haut zu bestimmen.

Dabei ist es wichtig, dass das Spektrometer nicht direkt reflektiertes Licht misst, sondern nur Licht, das bereits das Gewebe des untersuchten Körperteils durchlaufen hat. Daher muss die Lichtquelle gegenüber der Blende abgeschirmt sein, so dass nur aus dem Körperteil wieder austretendes Licht gemessen wird.

Das Beugungsbild der Blende liegt zum Beispiel so auf dem Kamerasensor, dass die Längsausdehnung der Blende der Y-Richtung entspricht und die dispersive Auffächerung in X-Richtung senkrecht dazu erfolgt. Dann entspricht jede Zeile des Kamerasensors einem Bildelement (oder einem Blendenlängenelement) des durch die Blende hindurchtretenden Lichts, dessen Spektrum in X-Richtung entlang einer Zeile des Kamerasensors verläuft. Jede Zeile bildet dann ein unabhängig gemessenes Spektrum. Jedem Spektrum ist ein Ort entlang der Längsausdehnung der Blende und somit eine Entfernung vom Einstrahlungsort zugeordnet. Mit zunehmender Entfernung vom Einstrahlungsort nimmt die durchlaufende Weglänge und -tiefe im Gewebe zu, d.h. die am nächsten zum Einstrahlungsort liegenden Austrittsorte haben Spektren entsprechend der kürzesten mittleren Weglänge und der niedrigsten erfassten Tiefe im Gewebe, während die am weitesten entfernte Austrittspunke Spektren haben, die auf Licht mit der größten zurückgelegten mittlere Weglänge und größten erreichten Tiefe im Gewebe zurückgehen. Durch separate Erfassung von Spektren, gegebenenfalls in einzelnen Intervallen entlang der Längsausdehnung der Blende zusammengefasst, und deren Auswertung mittels multivariater Analysen zur Ermittlung des jeweiligen Fettgehalts lässt sich so der Fettgehalt als Funktion der zurückgelegten mittleren Weglänge des Lichts und damit als Funktion der Tiefe im Gewebe bestimmen.

Es sind viele multivariable statistische Analyseverfahren bekannt, um Messdaten zu analysieren, zum Beispiel Korrelation, Regression, Varianzanalyse, Diskriminanz-Analyse und Hauptkomponenten- und Faktoranalyse. Im vorliegenden Fall ist es bevorzugt, die Spektren von reinem Fett, Wasser und Proteinen gespeichert bereitzuhalten und die erfassten Spektren unter Verwendung dieser bekannten Spektren einer Hauptkomponentenanalyse zu unterziehen, um den relativen Anteil von Fett zu bestimmen.

Unter dem Begriff Blende sollen hier alle optischen Mittel verstanden werden, die einen langgestreckten, streifenförmigen Bereich von austretendem Licht ausblenden, wobei der streifenförmige Bereich nicht notwendig in Längsrichtung durchgängig sein muss sondern auch aus einer reihenförmigen Folge von einzelnen separaten Bildelementen zusammengesetzt sein kann. Auch muss der langgestreckte streifenförmige Bereich nicht notwendig linear sein. In jedem Fall variiert bei einer vom Einstrahlungsort entfernt auf den Körperteil gerichteten Blende die Entfernung vom Einstrahlungsort als Funktion der Position entlang der Längsausdehnung der Blende. In einer bevorzugten Ausführungsform ist die Lichtquelle benachbart einem Ende einer linearen Blende angeordnet; in diesem Fall steigt die Entfernung vom Einstrahlungsort linear als Funktion der Länge entlang der Blende an. Es sind auch andere Anordnungen von Lichtquelle und Blenden möglich, die aber fest und vorherbestimmt sein müssen, damit vorab ein eindeutiger Zusammenhang zwischen der Entfernung vom Einstrahlungsort und der Position in der Blende hergestellt und gespeichert werden kann.

Zur Bestimmung des Fettanteils aus den Spektren als Funktion der Entfernung vom Einstrahlungsort und damit als Funktion unterschiedlich tiefer beteiligter Schichten unter der Haut können verschiedene Wege beschritten werden, um aus der Entfernungsabhängigkeit Informationen über die Tiefenabhängigkeit des Fettgehaltes zu bestimmen. In dem Artikel "Study of photon migration depths with time-resolved spectroscopy", W.Cui et al., Opt. Lett. 16, 1632-1634 (1991), wird ein Modell vorgeschlagen, wonach die durchschnittliche Tiefe T des Lichtweges ungefähr die Hälfte der Entfernung X zwischen Einstrahlungsort und dem Austrittsort beträgt: T = X/2. Betrachtet man nun den bestimmten Fettgehalt an einer Reihe von Austrittsorten Xᵢ, so lässt sich durch Vergleich der bestimmten Fettgehalte an verschiedenen Austrittsorten Xᵢ durch Umskalieren der Achse mit den Austrittsorten Xᵢ in eine entsprechende Achse mit der durchschnittlichen Tiefe Tᵢ gemäß der oben angegebenen Beziehung der Fettgehalt als Funktion der durchschnittlichen Tiefe des Lichtweges erhalten. Durch Differenzbildungen aufeinanderfolgender durchschnittlicher Tiefen Tᵢ lassen sich Informationen über den Fettgehalt in bestimmten Tiefenbereichen ermitteln. Durch Differenzieren der ermittelten Kurve des Fettgehaltes als Funktion der durchschnittlichen Tiefe nach der Tiefe lassen sich Aussagen über die Änderung des Fettgehaltes mit der Tiefe machen.

Es gibt auch elaboriertere Modelle für den Photonentransport in Materie und den Beitrag verschiedener Tiefen als Funktion der Entfernung vom Einstrahlungsort zum Austrittsort. Ein solches vollständigeres Modell ist zum Beispiel in der Dissertation "Nahinfrarotspektroskopie am Kopf des Erwachsenen mit Picosekunden-Zeitauflösung" von Jens Steinbrink beschrieben (URN: NBN:DE:KOBV:188-2000001281). Mit den aufgestellten Modellen lässt sich eine Entfaltung der Anteile verschiedener Tiefenbereiche zu den Spektren als Funktion der Entfernung Einstrahlungsort zum Austrittsort durchführen; eine solche Entfaltung wird dann in der Praxis mit Computerprogrammen vorgenommen, da die verwendeten Gleichungen zwar an sich mathematisch nicht kompliziert sind, aber aufgrund der vielen erforderlichen Rechenschritte in Matrizengleichungen am bequemsten mit dem Computer gelöst werden.

Eine andere Möglichkeit, aus der Entfernungsabhängigkeit die Tiefenabhängigkeit des Fettgehaltes zu bestimmen, besteht darin, eine größere Anzahl von Kalibrationskörpern mit bekanntem, aber unterschiedlichem Fettgehaltsverlauf als Funktion der Tiefe durchzumessen und die resultierenden Spektren als Funktion der Entfernung zwischen Einstrahlungsort und Austrittsort aufzuzeichnen. Durch Vergleich eines aktuell gemessenen Verlaufs des Fettgehalts als Funktion der Entfernung zwischen Einstrahlungsort und Austrittsort mit den entsprechenden bekannten Verläufen der Kalibrationskörper können dann Verläufe mit der besten Übereinstimmung ausgewählt oder als Linearkombination verschiedener bekannter Verläufe von Kalibrationskörpern dargestellt werden und daraus Aussagen über den Verlauf des Fettgehalts als Funktion der Tiefe gewonnen werden. Erfindungsgemäß erfolgt die dispersive Auffächerung des durch die Blende hindurchtretenden Lichts durch das Gitter in einer von der Längsrichtung der Blende verschiedenen Richtung; dabei kann die Richtung der spektralen Zerlegung senkrecht zur Längsrichtung der Blende stehen. Es kann aber auch jeder andere von 0 und 180° verschiedene Winkel sein; in diesem Fall steht die X-Achse (Richtung der spektralen Auffächerung) unter eben diesem Winkel zur Längsrichtung der Blende auf dem Kamerasensor. Jedem Punkt auf dem Kamerasensor kann dann eindeutig ein Wertepaar in dem (schrägwinkeligen) Koordinatensystem aus λ-Achse und Ort in Längsrichtung der Blende zugeordnet werden.

Vorzugsweise ist das Gitter des Spektrometers ein Blaze-Gitter, das dazu optimiert ist, das dispergierte Licht im Wellenlängenbereich von 700 bis 1.000 nm in einen vorgegebenen Winkelbereich konzentriert abzugeben. Klassische Gitter haben den Nachteil, dass das Licht einer bestimmen Wellenlänge hauptsächlich in die nullte Ordnung geht und der Rest über höhere Ordnungen verteilt wird, was einen Verlust an Helligkeit und damit Empfindlichkeit in jeder einzelnen Ordnung mit sich bringt. Dieser Nachteil wird durch die sogenannten Blaze-Gitter überwunden. Blaze-Gitter sind dazu optimiert, das Licht nur in eine bestimmte Richtung und somit bei gegebenem Wellenlängenbereich hauptsächlich in eine bestimmte Ordnung zu beugen. Dadurch kann ein Kamerasensor in dem optimierten Winkelbereich positioniert werden und braucht dort nur einen relativ kleinen Raumwinkelbereich zu überdecken. Zu den ersten Blaze-Gittern gehörten zum Beispiel Reflexionsgitter mit asymmetrisch sägezahnförmigen Oberflächengestaltungen, wobei die Sägezahnflanken als einzelne Spiegel jeweils so ausgerichtet sind, dass das Licht in Richtung der gewünschten Beugungsordnung reflektiert wird. Danach wurden auch holographische Gitter entwickelt, die den gleichen Effekt wie die zuerst beschriebenen Blaze-Gitter haben. Weiter Typen von Blaze-Gittern sind die später entwickelten sogenannten VPH-Gitter (volume phase holographic gratings). VPH-Gitter sind Transmissionsgitter, bei denen ein transparentes Material zwischen zwei Glas-oder Kunststoffscheiben eingeschlossen ist. In dem transparenten Material ist ein gewünschtes Muster eines variierenden Brechungsindex erzeugt, zum Beispiel durch holographische Belichtung und dadurch erfolgte Strukturänderung des Materials. Ein Beispiel für ein solches Blaze-Gitter ist in dem Artikel "Volume-Phase Holographic Gratings and the Efficiency of three Simple Volume-Phase Holographic Gratings", Samuel C. Barden et al., Publications of the Astronomical Society of the Pacific, Vol. 112, Seiten 809-820, Juni 2000, beschrieben. Mit solchen Blaze-Gittern lässt sich ein hoher Prozentsatz des gebeugten Lichts über den interessierenden Wellenlängenbereich in einen bestimmten Winkelbereich konzentrieren. Damit kann mit einem relativ kleinen Kamerasensor ein großer Anteil des Beugungsspektrums erfasst werden. Mit typischen Blaze-Gittern lassen sich ohne weiteres hohe Anteile von über 60% der Beugungsintensität in einem kleinen vorgegebenen Winkelbereich konzentrieren.

Vorzugsweise werden die Spektren bei der Analyse jeweils in Form ihrer weißnormierten Rohabsorptionsspektren und zusätzlich ihrer zweiten Ableitungen verwendet. Dieses macht das Verfahren unabhängig von gerätespezifischen Einflüssen wie z.B. Beleuchtungsschwankungen oder aber auch von nichtspezifischen breitbandigen Absorptionsunterschieden, welche im Körpergewebe von unterschiedlichen Personen vorliegen können.

Vorzugsweise hat die Vorrichtung einen transparenten Auflagekörper mit einer im Wesentlichen ebenen Oberfläche zur Auflage auf der Haut, durch den Licht von der Lichtquelle in das zu untersuchende Körperteil einstrahlbar ist, und einen weiteren transparenten Auflagekörper mit einer im Wesentlichen ebenen Oberfläche zu Auflage auf der Haut, durch den aus dem Körperteil wieder austretendes Licht, gegebenenfalls durch ein Objektiv, auf die Blende fällt, wobei die beiden transparenten Auflagekörper optisch voneinander getrennt sind, so dass kein direktes Licht von dem ersten Auflagekörper in den zweiten Auflagekörper gelangen kann. Diese Zwischenschaltung von transparenten Auflagekörpern mit ebenen Oberflächen zur Auflage auf der Haut hat den folgenden Vorteil. Mikroskopisch betrachtet weist die Haut erhebliche Unebenheiten oder Höhenvariationen auf. Wenn Licht aus dem Körperteil durch die Haut jeweils senkrecht zu der lokal betroffenen Hautoberfläche austritt, so variiert der Strahlungswinkel aufgrund der Unebenheiten von Punkt zu Punkt an der Haut und überstreicht daher insgesamt einen großen Bereich. Durch die Auflage des transparenten Auflagekörpers mit der ebenen Oberfläche werden die Unebenheiten der Haut reduziert und es erfolgt eine stärker gebündelte Ausstrahlung in den Auflagekörper. Umgekehrt hat der Auflagekörper für die Einstrahlung des Lichts aus der Lichtquelle in das Körperteil den Vorteil, dass das Licht durch die minimierten Unebenheiten auf der Hautoberfläche stärker gebündelt in den zu untersuchenden Körperteil eingestrahlt werden kann. Das Licht der Lichtquelle sollte ein Wellenlängenbereich von 700 nm bis 1.000 nm einschließen, da dieser Wellenlängenbereich, wie oben schon erläutert, eine gute Eindringtiefe in das Gewebe ermöglicht, und andererseits die Bereiche umfasst, in denen Fett und Wasser charakteristische Unterschiede aufweisen.

Wie schon bemerkt, darf das Licht der Lichtquelle nicht direkt von der Hautoberfläche in das Spektrometer reflektiert werden, sondern muss durch das Gewebe wandern (Transmission mit diffusen Streuvorgängen).

Die Beleuchtungseinheit kann konventionell mit einer thermischen Lichtquelle (z.B. Halogenlampe) ausgerüstet sein. Bevorzugt wird aber eine Lichtquelle, die auf LEDs basiert. Damit der Spektralbereich von 800nm bis 1000nm kontinuierlich und homogen ausgeleuchtet werden kann, müssen LEDs mit unterschiedlichen Peakwellenlängen kombiniert und in einem symmetrischen Raster verteilt werden und deren abgestrahltes Licht durch bekannte Verfahren gemischt oder überlagert erden. Eine andere Möglichkeit ist der Einsatz einer LED-Sorte mit kürzerer Wellenlänge, die mit einem breitbandigen Fluoreszenzfarbstoff überzogen wurde, der in dem geforderten Spektralbereich die notwendige kontinuierliche Strahlung erzeugt. Wichtig ist, dass die Strahlung intensiv, breitbandig, temperaturstabil und möglichst in schnellen Pulsen erzeugbar ist. Da Halogenlampen abgesehen von der breitbandigen Beleuchtung diese Kriterien nicht erfüllen, sind die LED-Varianten eindeutig zu bevorzugen.

Da das Spektrometer ein zweidimensionales Spektrometer ist, lassen sich die Spektren ortsaufgelöst erfassen. So sind die Spektren alle von jeweils einem Ort entlang der Längsausdehnung des ausgeblendeten schlitzförmigen Bereichs aufgenommen, so dass jedem Spektrum der zugehörige Ort und damit eine Entfernung vom Einstrahlungsort zugeordnet werden kann. Das entlang des schlitzförmigen ausgeblendeten Bereichs austretende Licht hat abhängig vom Austrittsort unterschiedliche Weglängen durch das Gewebe zurückgelegt. Mit zunehmender Weglänge durch das Gewebe werden immer tiefere Bereiche des Gewebes einbezogen. Mittels dieser weglängenabhängigen spektralen Information lässt sich eine ortsaufgelöste und mathematisch transformiert eine tiefenabhängige Information bezüglich des Fettgehalts herleiten.

Da die Lichtintensitäten mit größerer Entfernung zwischen Einstrahlungsort und Austrittsort stark abnehmen, kann es zweckmäßig sein, mehrere Aufnahmen mit unterschiedlichen Belichtungszeiten und Verstärkungseinstellungen des Kamerasensors aufzunehmen und in der Auswertung zu kombinieren.

Das Spektrometer kann beispielsweise drei Objektive aufweisen. Das erste erzeugt eine normale zweidimensionale Abbildung des aus dem beobachteten Körperteil nach Transmission und diffuser Streuung wieder austretenden Lichts. In der Bildebene des ersten Objektivs ist die spaltförmige Blende angeordnet. Die spaltförmige Blende selektiert zum Beispiel einen 30 µm breiten Streifen aus dem Bild. Das zweite Objektiv bildet die spaltförmige Blende ins Unendliche ab. Hinter dem zweiten Objektiv liegt das Gitter, das eine dispersive Aufspaltung des Lichts in Richtung senkrecht zur Längsausdehnung der spaltförmigen Blende bewirkt. Das letzte Objektiv macht wieder die Abbildung vom Unendlichen auf das reale Bild, welches nun aber durch das zwischenliegende Gitter spektral aufgefächert ist und durch den Kamerasensor aufgenommen wird.

Bei dem erfindungsgemäßen Verfahren wird aus dem Licht, das aus dem Körperteil entfernt von dem Einstrahlungsort wieder austritt, ein streifenförmiger Bereich so ausgeblendet, dass die Entfernung vom Einstrahlungsort zum Austrittsort entlang des streifenförmigen Bereichs von dem Ende, das dem Einstrahlungsort zugewandt ist, entlang der Länge des streifenförmigen Bereichs zunimmt. Dies wird am besten gewährleistet, wenn der Einstrahlungsort in der Verlängerung der Längsrichtung des streifenförmigen Bereichs liegt. Eine Entfernungsabhängigkeit von Austrittsort im streifenförmigen Bereich und Einstrahlungsort ist zwar auch gegeben, wenn der Einstrahlungsort außerhalb der Verlängerung der Längsrichtung des streifenförmigen Bereichs liegt, die Korrelation zwischen Austrittsort entlang des streifenförmigen Bereichs und der Entfernung zum Einstrahlungsort ist jedoch bei der zuerst genannten Anordnung am besten. Anschließend wird durch den streifenförmigen Bereich fallendes Licht einer Beugung an einem Gitter so unterzogen, dass die dispersive Auffächerung des durch den streifenförmigen Bereich durchgetretenen Lichts in einer von der Längsrichtung des streifenförmigen Bereichs verschiedenen Richtung, vorzugsweise senkrecht dazu, erfolgt. Dieses Beugungsbild des Gitters mit der Längsrichtung des streifenförmigen Bereichs in der ersten Richtung und der dispersiven spektralen Auffächerung des Lichtes in einer davon verschiedenen zweiten Richtung wird von einem Kamerasensor aufgenommen. Das aufgenommene Beugungsbild wird dann als Vielzahl von Spektren, jedes einem Austrittsort entlang der Längsrichtung des streifenförmigen Bereichs zugeordnet, ausgewertet. Bei senkrechter Ausrichtung von erster und zweiter Richtung kann jede Kamerasensorzeile einem Austrittsort des Lichts in dem streifenförmigen Bereich entsprechen, wobei die spektrale Auflösung dann entlang der Kamerazeile vorliegt. Die Anzahl der Spektren entspricht dann der Zeilenanzahl des Kamerasensors. Jedem Spektrum kann der zugehörige Austrittsort entlang der Längsrichtung des ausgeblendeten streifenförmigen Bereichs zugeordnet werden, wonach die Spektren multivariaten statistischen Analysen unterzogen werden, um die jeweiligen Fettgehalte aus den Spektren in Zuordnung zur Entfernung zwischen Eintrittsort und jeweiligem Austrittsort und damit als Funktion der Tiefe der beteiligten Gewebeschichten unter der Haut zu bestimmen.

Die Erfindung wird im Folgenden anhand von Ausführungsbeispielen in den Zeichnungen beschrieben, in denen:
Fig. 1 eine schematische Prinzipdarstellung zum Aufbau der Vorrichtung und zur Verfahrensweise zur Bestimmung des Fettgehaltes zeigt,
Fig. 2 eine schematische Draufsicht auf die Vorrichtung aus Fig. 1 von unten zeigt,
Fig. 3 schematisch den Aufbau einer Vorrichtung zur Bestimmung des Fettgehaltes zeigt,
Fig. 4 eine Prinzipskizze zur Erläuterung der Funktionsweise der Vorrichtung aus Fig. 3 zeigt,
Fig. 5 Spektren (mit erster und zweiter Ableitung) von reinem Wasser und von reinem Fett zeigt,
Fig. 6 aufgenommene Spektren (zweite Ableitung) von verschiedenen Personen zeigt,
Fig. 7 aufgenommene Spektren (zweite Ableitung) für verschiedene Entfernungen von Einstrahlungsort zum Austrittsort zeigt, wobei die Entfernungen in entsprechende mittlere Tiefen der erfassten Schichten unter der Haut umgerechnet sind,
Fig. 8 den ermittelten Fettgehalt als Funktion der mittleren Tiefe der beteiligten Schichten unter der Haut für eine dicke und eine dünne Person zeigt, und

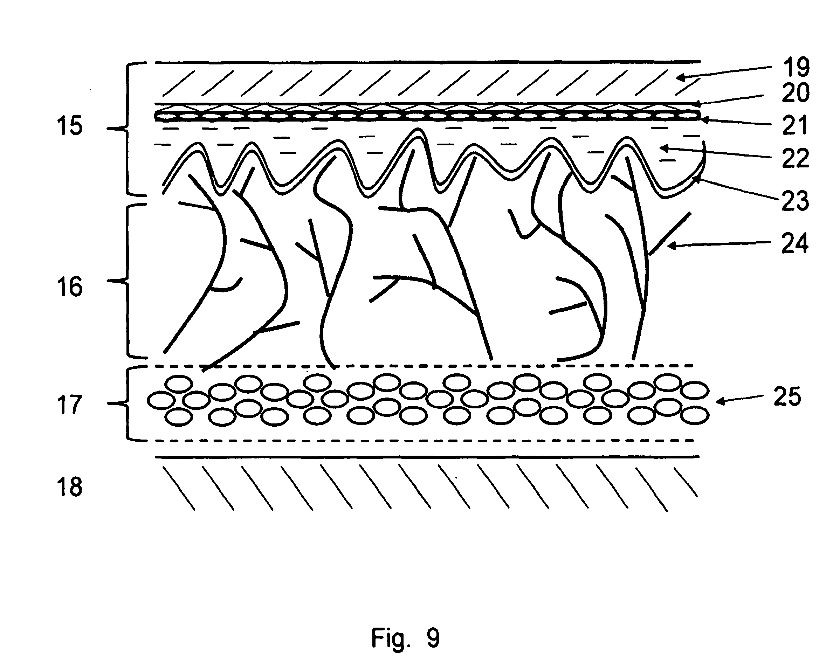

Fig. 9 eine schematische Darstellung der Gewebestruktur eines Körperteils zeigt.

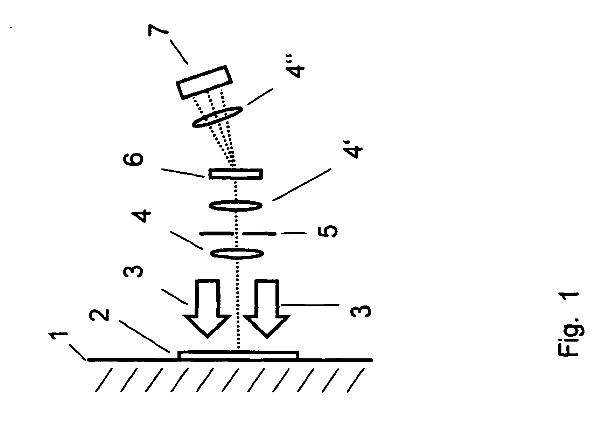

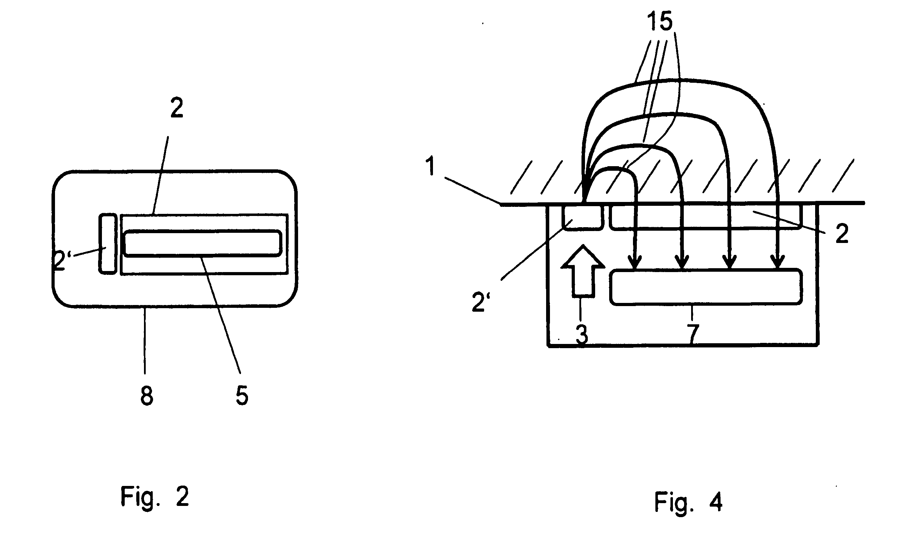

Fig. 1 zeigt als schematische Prinzipdarstellung den Aufbau einer erfindungsgemäßen Vorrichtung. Das zu untersuchende Körperteil ist mit 1 bezeichnet. Auf der Haut des zu untersuchenden Körperteils 1 liegt ein transparenter Auflagekörper 2 mit einer ebenen Oberflächen auf. Lichteinstrahlung erfolgt gegenüber der Zeichnungsebene senkrecht versetzt in einem Bereich jenseits des Auflagekörpers 2 durch einen weiteren entsprechenden transparenten Auflagekörper 2' (Fig. 2). Die Auflagekörper 2, 2' dienen dazu, Hautunebenheiten zu minimieren und dadurch eine bessere Einkopplung bzw. Auskopplung von Licht zu ermöglichen. Die transparenten Auflagekörper sollen so gegeneinander abgeschirmt sein, dass kein Licht direkt aus dem Auflagekörper 3' für die Lichtquelle in den Auflagekörper 2 übertreten kann und dass direkt von der Hautoberfläche reflektiertes Licht nicht in den Auflagekörper 2 gelangt.

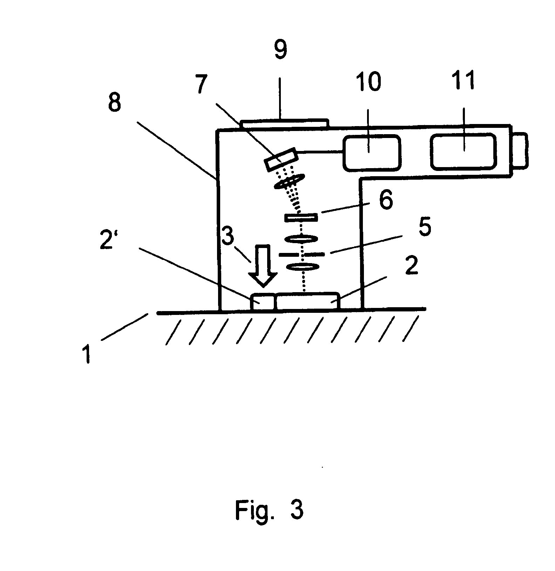

Die Lichtquelle 3 erzeugt Licht einschließlich des Spektralbereichs 800 nm bis 1.000 nm, der einerseits eine relativ hohe Eindringtiefe in das Gewebe gewährleistet und andererseits charakteristische unterschiedliche Merkmale insbesondere zwischen Wasser und Fett umfasst. Das durch die Lichtquelle 3 über den transparenten Auflagekörper 2' (Fig. 3) eingekoppelte Licht wird durch Transmission und Transflexion im Gewebe weitergeleitet und Teile dieses Lichts treten dann an einem Ort entfernt von dem Einstrahlungsort wieder aus dem Körperteil aus und in den Auflagekörper 2 ein. Dieses durch das Gewebe transmittierte Licht ist für die nachfolgend beschriebene spektroskopische Bestimmung des Fettgehaltes geeignet, denn genaue Messungen, die auf den Fettgehalt sensitiv sind, können nur im Gewebe gewonnen werden, während eine Reflexionsmessungen keine genaue quantitative Bestimmung des Fettgehaltes, insbesondere keine tiefenabhängige, erlauben. Durch die Auswahl des Spektralbereichs im Bereich von 800 nm bis 1.000 nm kann hier mit großen Weglängen der Transmission vom Einstrahlungsort zum Austrittspunkt gearbeitet werden, da das Gewebe in diesem Spektralbereich relativ transparent ist. Durch die konzentrierte Einkopplung des Lichts der Lichtquelle kann der Einstrahlungsort hinreichend genau festgelegt werden, so dass auch eine Entfernung zwischen Einstrahlungsort und Austrittspunkt hinreichend genau festlegbar ist.

Über dem Auflagekörper 2 ist eine spaltförmige Blende 5 angeordnet, deren Längsrichtung, in der Darstellung von Fig. 1, senkrecht zur Figurenebene steht. Mit dieser spalt- oder schlitzförmigen Blende 5 wird ein schlitzförmiger Bereich aus dem durch das Gewebe transmittierten Lichts ausgeblendet, wobei der Austrittsort oder der Ort entlang der spaltförmigen Blende 5 jeweils eine zugeordnete Entfernung zum Einstrahlungsort hat. Der von der Blende 5 ausgeblendete streifenförmige Bereich des aus dem Körperteil ausgetretenen und über ein erstes Objektiv 4 abgebildeten Lichts wird über ein zweites Objektiv 4' auf ein Gitter 6 geworfen. Dieses Gitter 6 ist so aufgebaut und angeordnet, dass die dispersive, wellenlängenabhängige Auffächerung des gebeugten Lichts in einer von der Längsausdehnung der Blende verschiedenen, vorzugsweise dazu senkrechten, Richtung erfolgt. In der Darstellung von Fig. 1 bedeutet dies, dass die Längsrichtung der Blende senkrecht zur Figurenebene steht, während die dispersive Auffächerung des Lichts in der Figurenebene erfolgt. Über ein weiteres Objektiv 4" wird das Beugungsbild der Blende dann auf einen Kamerasensor 7 geworfen. Auf der Sensoroberfläche verläuft dann das Beugungsbild der Blende mit der Längsausdehnung der Blende in einer ersten Richtung (in dem dargestellten Beispiel senkrecht zur Figurenebene) und mit der dispersiven Auffächerung des Beugungsbildes in einer zweiten, hier zur ersten senkrechten Richtung (in der Figurenebene). Auf der Kamerasensoroberfläche entspricht dann jede Kamerasensorzeile einem Bildelement entlang der Längsausrichtung der spaltförmigen Blende 5, und entlang der Kamerasensorzeile liegt die spektrale Auffächerung dieses Bildelements vor. Durch die aufeinanderfolgenden Kamerasensorzeilen wird also eine aufeinanderfolgende Abfolge von Spektren erfasst, die einer Abfolge von Bildelementen entlang der Längsausdehnung der spaltförmigen Blende 5 mit zunehmender Entfernung vom Einstrahlungsort entsprechen.

Bei dem Kamerasensor 7 handelt es sich vorzugsweise um einen zweidimensionalen CMOS-Digitalkamerasensor. Dieser ermöglicht es, mit einer einzigen Bildaufnahme gleichzeitig bis zu 1.000 Spektren (ein Spektrum pro Zeile) mit einer Datentiefe von bis zu 12 Bit aufzunehmen. Jedes der Spektren entspricht damit dem Spektrum eines Bildelementes der Blende, d.h. man kann sich die schlitzförmige Blende 5 in 1.000 Bildelemente unterteilt denken, wobei das Licht jedes dieser Bildelemente durch das Gitter dann auf eine Zeile des Kamerasensors abgebildet wird. Der Kamerasensor kann die Bildaufnahme etwa 50-mal pro Sekunde wiederholen. Durch den zur Analyse benötigten kleinen Spektralbereich kann die bei diesen Sensoren mögliche Teilbildaufnahme eingesetzt werden. Bei CMOS-Sensoren können Teilbilder (regions of interest - ROI) eingestellt werden, die es ermöglichen, nur den eingestellten interessierenden Bildbereich des Sensors bei gleichzeitiger Beibehaltung der Grunddatenrate auszulesen. Dieses Verfahren erlaubt es, über 20.000 Spektren in weniger als einer Sekunde aufzunehmen.

Fig. 3 zeigt eine schematische Prinzipdarstellung des Aufbaus einer erfindungsgemäßen Vorrichtung, die als Handgerät ausgeführt sein kann. Die Vorrichtung hat ein Gehäuse 8, das mit seiner Unterseite in Auflage zum Beispiel mittig auf den Bizeps gebracht werden kann. In der Auflagefläche sind die transparenten Auflagekörper 2 und 2' gehalten. Durch den transparenten Auflagekörper 2' wird Licht aus der Lichtquelle 3 in das Körperteil 1 eingekoppelt. Diffus gestreutes Licht pflanzt sich durch das Gewebe des Körperteils 1 fort, und ein Teil des Lichts tritt jenseits des einkoppelnden Auflagekörpers 2' im Bereich des Auflagekörpers 2 aus dem Körperteil wieder aus und in den transparenten Auflagekörper ein. Die zuvor schon im Zusammenhang mit Fig. 1 beschriebene Spektrometereinrichtung erzeugt dann das Beugungsbild auf der Kamerasensorfläche mit der spektralen Auffächerung entlang einer ersten Richtung, zum Beispiel entlang der Kamerasensorzeilen, und einer Ortsabbildung der Blende entlang der aufeinanderfolgenden Kamerasensorzeilen. Dieses Beugungsbild der Blende wird in der Datenverarbeitungseinrichtung 10, die neben anderen elektrischen Verbrauchern von einem Akkumulator 11 versorgt wird, verarbeitet. Ergebnisse der Bestimmungen der Fettgehalte können auf eine Anzeige 9 zur Anzeige gebracht werden.

Die Detailansicht in Fig. 4 verdeutlicht noch einmal die Funktionsweise der Vorrichtung aus Fig. 3. Licht aus der Licht-quelle 3 wird über den Auflagekörper 2' eingekoppelt und pflanzt sich dort durch Transmission und Transflexion fort. Durch die Ausblendung (die Blende ist in Fig. 4 nicht gezeigt) eines streifenförmigen Bereichs, dessen Längsausdehnung in dieser Darstellung in der Figurenebene liegt, wird ein streifenförmiger Bereich definiert, bei dem die Position der Längsrichtung des streifenförmigen Bereichs mit der von dem Einstrahlungspunkt zum Austrittspunkt zurückgelegten Weglänge korreliert. Mit steigender Weglänge 15 und damit mit steigender Entfernung entlang der Längsrichtung des ausgeblendeten streifenförmigen Bereichs vom Einstrahlungsort steigt auch die Tiefe der an den Streuvorgängen beteiligten Gewebeschichten. Damit können den Bildelementen des ausgeblendeten streifenförmigen Bereichs zurückgelegte Weglängen im Gewebe und erfasste Tiefen im Gewebe zugeordnet werden und damit auch eine tiefenabhängige Bestimmung von Fettgehalten vorgenommen werden.

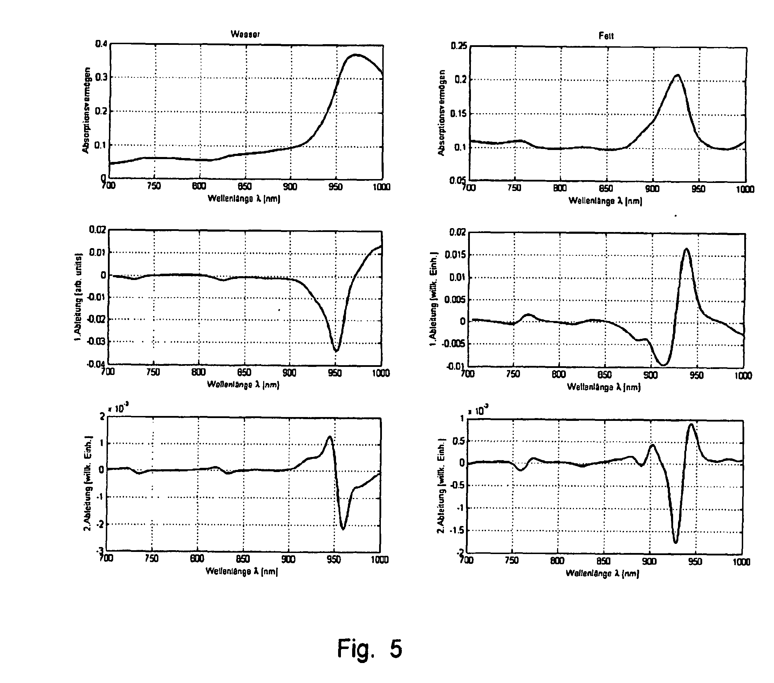

Fig. 5 zeigt die Einzelspektren von reinem Wasser und Fett im Wellenlängenbereich von 700 bis 1.000 nm, wobei neben dem Absorptionsvermögen (=log(I(λ)/Iᵢₙ(λ)) auch die ersten und zweiten Ableitungen der relativen Intensität I(λ)/Iᵢₙ(λ) dargestellt sind, wobei I(λ) die gemessenen Intensität wieder austretender Strahlung und Iᵢₙ(λ) die Intensität der einfallenden Strahlung ist. Im Zusammenhang mit der vorliegenden Erfindung wird vorzugsweise mit den Spektren in Form von deren zweiten Ableitungen d²(I(λ)/Iᵢₙ(λ))/dλ² gearbeitet oder mit einer kombinierten Auswertung von Absorptionsrohspektren und der 2ten Ableitung. Wie aus Fig. 5 ersichtlich zeigen Wasser und Fett in ihren Spektren in zweiter Ableitung charakteristische Unterschiede, zum Beispiel zeigt Fett im Bereich von etwa 930 nm ein ausgeprägtes Minimum, während Wasser dort in der zweiten Ableitung einen relativ hohen positiven Wert hat. Aufgrund dieser und anderer Unterschiede können an gemessene Spektren die vorab bestimmten Einzelspektren reiner Substanzen angepasst werden. Dazu wird ein multivariates statistisches Analyseverfahren verwendet, um die Beiträge der Einzelsubstanzen zu dem erfassten Spektrum zu ermitteln und daraus insbesondere den relativen Beitrag des Fetts zu bestimmen. Anschaulich wird in einem solchen Analyseverfahren das gemessene Spektrum als Linearkombination der Einzelspektren der reinen Substanzen dargestellt und die Koeffizienten der Einzelspektren in derjenigen Linearkombination der Einzelspektren ermittelt, die die beste Übereinstimmung mit dem gemessenen Spektrum liefert. Es sollten daher Einzelspektren aller Substanzen vorab gemessen und gespeichert werden, die in dem untersuchten Wellenlängenbereich relevant sind. Soweit die zweiten Ableitungen der Spektren betrachtet werden, müssen natürlich nur solche Substanzen berücksichtigt werden, deren Spektren in zweiter Ableitung in dem fraglichen Wellenlängenbereich von 0 verschieden sind. Zum Beispiel brauchen Melanin und Hämoglobin in dem hier vorzugsweise betrachteten Spektralbereich von 800 nm bis 1.000 nm nicht berücksichtigt zu werden, da sie keine signifikanten Steigungsänderungen in den Spektren aufweisen und in der zweiten Ableitung der Spektren näherungsweise 0 sind. Bei der Aufnahme der Einzelspektren ist insbesondere beim Wasserspektrum auf dessen Temperaturabhängigkeit zu achten, d.h. die Einzelspektren sollten bei einer der Körpertemperatur entsprechenden Temperatur aufgenommen werden.

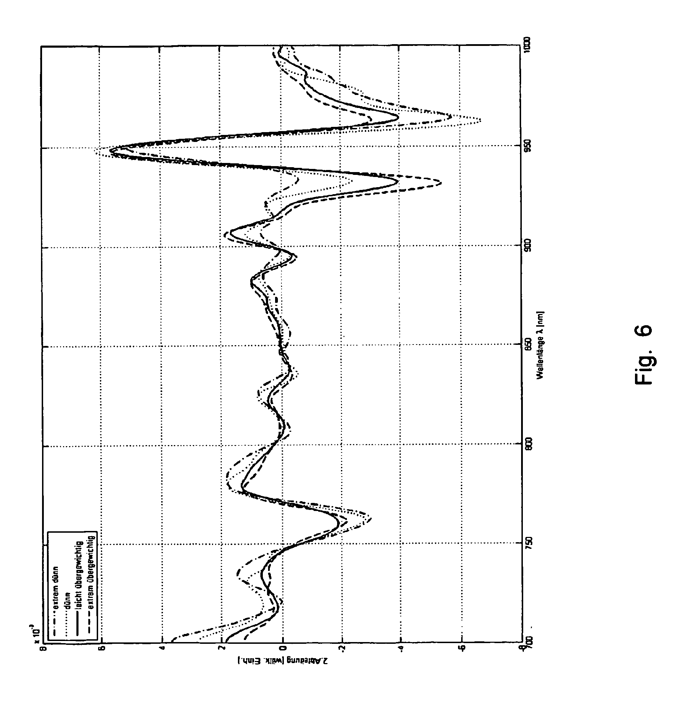

Fig. 6 zeigt erfasste Spektren (zweite Ableitungen) für verschiedene Personen, und zwar für eine extrem dünne, eine dünne, eine leicht übergewichtige und eine extrem übergewichtige Person. Es ist deutlich zu erkennen, dass sich zum Beispiel in den Wellenlängenbereichen um 925 nm, um 945 nm und 960 nm signifikante Unterschiede beobachten lassen.

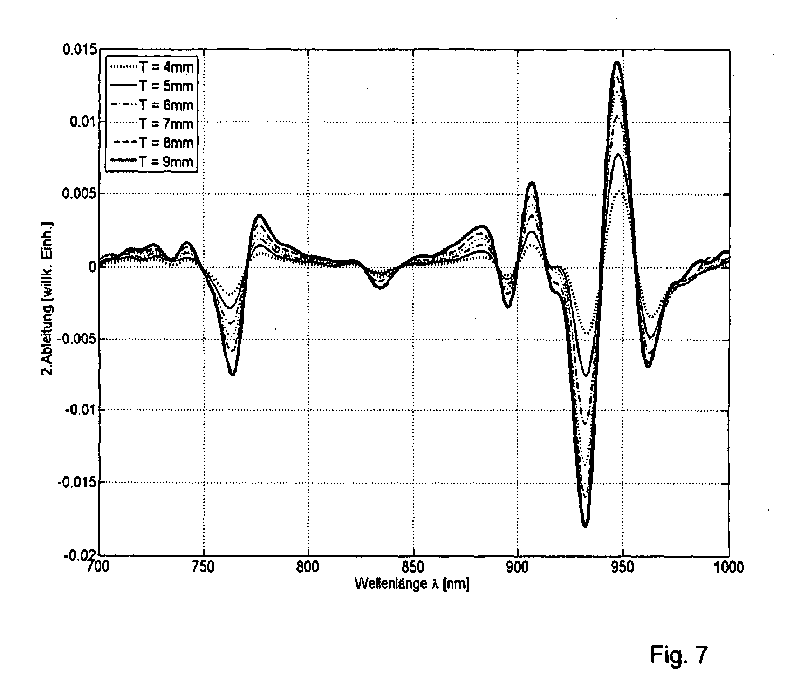

Fig. 7 zeigt Spektren (zweite Ableitungen) für verschiedene mittlere Tiefen der erfassten Schichten unter der Haut. Bei der Umrechung von der Entfernung des Austrittsortes des Lichts vom Einstrahlungsort in die mittlere Tiefe der beteiligten Gewebeschichten wurde das oben zitierte Modell verwendet, wonach die durchschnittliche Tiefe T der Hälfte der Entfernung X zwischen Einstrahlungsort und dem Austrittsort entlang der Blende beträgt: T = X/2. Die entsprechenden mittleren Tiefen sind in dem Diagramm angegeben. Qualitativ lässt sich daraus ablesen, dass der Fettgehalt mit zunehmender Tiefe T abnimmt, was daraus ersichtlich ist, dass das Minimum bei etwa 935 nm und das darauf folgende Maximum bei etwa 945 nm mit zunehmender Tiefe weniger ausgeprägt sind. Wie ein Vergleich mit Fig. 5 in dem rechten Diagramm unten zeigt, ist insbesondere das genannte Minimum charakteristisch für Fett. Die in Fig. 7 dargestellten Spektren stammen von einer dicken Person, bei der der Fettgehalt mit zunehmender Tiefe der beteiligten Schichten unter der Haut eher zunimmt.

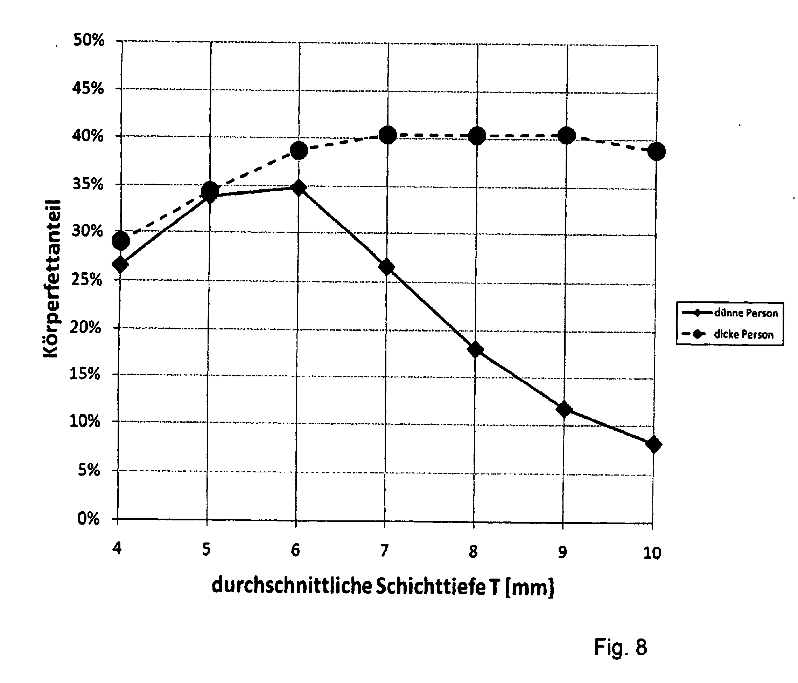

Durch Auswertung von Spektren wie den in Fig. 7 gezeigten, kann für verschiedene durchschnittliche Tiefen T jeweils der zugehörige Fettgehalt bestimmt werden. In Fig. 8 sind so bestimmte Fettgehalte als Funktion der durchschnittlichen Tiefe der erfassten Gewebeschichten unter der Haut dargestellt. Dabei sind in Fig. 8 die so bestimmten Fettanteile beispielhaft für eine dünne Person und eine dicke Person aufgetragen. Bei der dünnen Person ist zunächst ein leichter Anstieg des Fettanteils zu beobachten, der einer kleinen subkutanen Fettschicht zuzuordnen ist. In größeren Tiefen nimmt dann bei der dünnen Person der Fettgehalt ab; dies entspricht dem zunehmenden Beitrag von Muskelgewebe in größeren Tiefen, die sehr wenig Fett enthalten. Bei der dicken Person ist diese Abnahme des Fettgehalts in größeren durchschnittlichen Tiefen T nicht zu beobachten, was einem höheren Fettanteil auch in tieferliegendem Muskelgewebe bei der übergewichtigen Person entspricht. Fig. 8 zeigt schematisch den Gewebeaufbau eines Körperteils. Die Haut wird in drei Gebiete eingeteilt: Die oberste Schicht ist die Epidermis (Oberhaut 15), darunter liegt die Dermis (Lederhaut 16) und darunter die Subcutis (Unterhaut 17). Die äußerste Schicht der Epidermis 23 ist die Hornschicht (stratum corneum) darunter liegt die Glanzschicht (stratum lucidum), darunter die Körnerzellschicht (stratum granulosum), darunter die Stachelzellschicht (stratum spinosum) und darunter die Basalzellschicht (stratum basalec). In der Dermis 16 befinden sich hauptsächlich die Blutgefäße 24, aber auch Talgdrüsen, Haarfolikel, Muskeln und Schweißdrüsen. In der Subcutis 17 befindet sich das Bindegewebe mit den subcutanen Fettzellen 25. Unter der Haut liegen dann die Faszienmuskeln, Sehnen und Knochen 18. Im Bereich des Bizeps und für die Fettmessung relevant liegt hier das Muskelgewebe und erst sehr viel tieferliegend der Knochen, der von der Messung im Allgemeinen nicht mehr erfasst wird. Daher kann hier bei einer tiefenaufgelösten Messung ein Bereich der Dermis mit geringerem Fettanteil erwartet werden, dann in der Subcutis die Fettschicht mit einem hohen Fett und niedrigen Wasseranteil und danach das Muskelgewebe mit einem variierenden Fettanteil. Dieser letztere ist besonders relevant, da dieser Fettanteil in Korrelation mit dem Fettanteil im Organgewebe steht.

## Patentansprüche

1. Vorrichtung zur Bestimmung des Fettgehaltes des menschlichen Körpers mit einer Lichtquelle (3) zur Einstrahlung von Licht, das einen Anteil im NIR-Spektralbereich einschließt, in einen Körperteil, einer Lichtsensoreinrichtung (7) zur Aufnahme von aus dem Körperteil entfernt von dem Einstrahlungsort auf derselben Seite des Körperteils wieder austretendem Licht, einer Spektrometereinrichtung zur differenzierten Erfassung von wenigstens zwei Spektralbereichen des wieder austretenden Lichts und mit einer Datenverarbeitungseinheit (10), die Messsignale der Lichtsensoreinrichtung aufnimmt und zur Bestimmung des Fettgehaltes des Körperteils verarbeitet, **dadurch gekennzeichnet, dass** die Spektrometereinrichtung eine schlitzförmige Blende (5) zur Ausblendung eines schlitzförmigen Bereichs des aus der Körperstelle entfernt von dem Einstrahlungsort wieder austretenden Lichts, wobei die Blende so zur Lichtquelle angeordnet ist, dass die Entfernung zum Einstrahlungsort ausgehend von dem dem Einstrahlungsort zugewandten Ende entlang der Blende variiert, und ein Gitter (6) aufweist, das so aufgebaut und angeordnet ist, dass die dispersive Auffächerung des durch die Blende (5) hindurchtretenden Lichts in einer von der Längsrichtung der Blende verschiedenen Richtung erfolgt, dass die Lichtsensoreinrichtung (7) in Form eines Kamerasensors so aufgebaut und angeordnet ist, dass er ein Beugungsbild des Gitters (6) mit der Längsrichtung der Blende in einer ersten Richtung und mit der dispersiven spektralen Auffächerung des Lichtes in einer von der ersten verschiedenen, zweiten Richtung auf seiner Kamerasensorfläche aufnimmt, und dass die Datenverarbeitungseinheit weiter dazu vorbereitet ist, die von dem Kamerasensor (7) empfangenen Signale als eine Vielzahl von Spektren entsprechend einer Vielzahl von Bildelementen entlang der Längsrichtung der Blende aufzunehmen und die Vielzahl von Spektren in Zuordnung zu dem Ort entlang der Längsausdehnung der Blende, von dem das jeweilige Spektrum stammt, multivariaten statistischen Analysen zu unterziehen, um die jeweiligen Fettgehalte aus den Spektren in Zuordnung zur Entfernung zwischen Eintritts- und Austrittsort, die das Licht des jeweiligen Spektrums zurückgelegt hat, und damit als Funktion der Tiefe der beteiligten Schichten unter der Haut zu bestimmen.

2. Vorrichtung nach Anspruch 1, **dadurch gekennzeichnet, dass** das Gitter (6) des Spektrometers ein Blaze-Gitter ist, das dazu optimiert ist, das dispergierte Licht im Wellenlängenbereich von 700 bis 1.000 nm in einen vorgegebenen Winkelbereich konzentriert abzugeben, das Blaze-Gitter ein Reflexions- oder Transmissionsgitter mit asymmetrisch sägezahnförmig gestalteter Oberfläche oder ein holographisches Gitter ist.

3. Vorrichtung nach Anspruch 2, **dadurch gekennzeichnet, dass** das Blaze-Gitter ein VPH-Gitter (volume phase holographic grating) ist.

4. Vorrichtung nach einem der vorhergehenden Ansprüche, **dadurch gekennzeichnet, dass** die Datenverarbeitungseinheit (10) Spektren von reinem Körperfett, Wasser und Proteinen gespeichert enthält und weiter dazu vorbereitet ist, nach einem Verfahren der multivariaten Statistik die erfassten Spektren aus den Anteilen der gespeicherten Einzelspektren der genannten Substanzen zusammenzusetzen und daraus den relativen Gehalt an Körperfett zu be stimmen.

5. Vorrichtung nach einem der vorhergehenden Ansprüche, **dadurch gekennzeichnet, dass** die Lichtquelle (3) und die Blende (5) so zueinander angeordnet sind, dass die Lichtquelle Licht nahe an einem Ende der schlitzförmigen Blende Licht abstrahlt, so dass die Entfernung vom Einstrahlungsort zu einem Punkt entlang der Länge der Blende von dem benachbart der Lichtquelle liegendem Ende aus linear zunimmt.

6. Vorrichtung nach einem der vorhergehenden Ansprüche, **dadurch gekennzeichnet, dass** die Datenverarbeitungseinheit (10) dazu vorbereitet ist, die Längsausdehnung der Blende in eine Mehrzahl von Intervallen zu unterteilen und die Spektren in jedem Intervall zusammengefasst zur Bestimmung des Fettgehalts zu analysieren, jedem Intervall eine Entfernung vom Einstrahlungsort zuzuordnen und die Fettgehalte in den einzelnen Intervallen nach einem vorgegebenem Modell in Fettgehalte als Funktion der Tiefe der beteiligten Gewebeschichten unter der Haut umzurechnen.

7. Vorrichtung nach Anspruch 6, **dadurch gekennzeichnet, dass** die Datenverarbeitungseinheit (10) dazu vorbereitet ist, jedem Intervall, dessen Entfernung vom Einstrahlungsort Xᵢ beträgt, die mittlere Tiefe Tᵢ der beteiligten Gewebeschichten unter der Haut gemäß Tᵢ=Xᵢ/2 zuzuordnen.

8. Vorrichtung nach einem der vorhergehenden Ansprüche, **dadurch gekennzeichnet, dass** sie einen ersten transparenter Auflagekörper (2') mit einer im Wesentlichen ebenen Oberfläche zur Auflage auf der Haut, durch den Licht von der Lichtquelle (3) in den zu untersuchenden Körperteil einstrahlbar ist, und einen zweiten transparenten Auflagekörper (2) mit einer im Wesentlichen ebenen Oberfläche zur Auflage auf der Haut aufweist, durch den aus dem Körperteil wieder austretendes Licht auf die Blende (5) fällt, wobei die beiden transparenten Auflagekörper (2,2') optisch voneinander getrennt sind, so dass kein direktes Licht aus dem ersten Auflagekörper (2') in den zweiten Auflagekörper (2) gelangen kann.

9. Vorrichtung nach Anspruch 8, **dadurch gekennzeichnet, dass** sie die ebenen Auflageflächen des ersten (2') und zweiten (2) transparenten Auflagekörpers im Wesentlich in einer Ebene angeordnet sind.

10. Verfahren zur Bestimmung des Fettgehaltes eines Körperteils eines menschlichen Körpers, bei dem Licht, das einen Anteil im NIR-Spektralbereich aufweist, aus einer Lichtquelle (3) in den Körperteil (1) eingestrahlt wird, auf derselben Seite des Körperteils entfernt von dem Einstrahlungspunkt wieder daraus austretendes Licht mit einer Lichtsensoreinrichtung (7) erfasst wird, wobei eine Spektrometereinrichtung zur differenzierten Erfassung von wenigstens zwei Spektralbereichen des wieder austretenden Lichts vorgesehen ist, und bei dem die Messsignale der Lichtsensoreinrichtung (7) von einer Datenverarbeitungseinheit (10) aufgenommen und zur Bestimmung des Fettgehaltes des Körperteils verarbeitet werden, **dadurch gekennzeichnet,**
**dass** aus Licht, das aus dem Körperteil (1) entfernt von dem Einstrahlungsort wieder austritt, ein streifenförmiger Bereich so ausgeblendet wird, dass die Entfernung vom Einstrahlungsort zum Austrittsort entlang des streifenförmigen Bereichs von dem dem Einstrahlungsort zugewandten Ende des streifenförmigen Bereichs aus entlang der Länge des streifenförmigen Bereichs zunimmt,
**dass** das durch den streifenförmigen Bereich fallende Licht einer Beugung an einem Gitter (6) so unterzogen wird, dass die dispersive Auffächerung des durch den streifenförmigen Bereich durchgetretenen Lichts in einer von der Längsrichtung des streifenförmigen Bereichs verschiedenen Richtung erfolgt,
**dass** das Beugungsbild des Gitters mit der Längsrichtung des streifenförmigen Bereichs in einer ersten Richtung und mit der dispersiven spektralen Auffächerung des Lichtes in einer von der ersten verschiedenen zweiten Richtung mit einem Kamerasensor (7) aufgenommen wird und
**dass** das aufgenommene Beugungsbild als Vielzahl von Spektren entlang der Längsrichtung des streifenförmigen Bereichs ausgewertet wird, wobei jedem Spektrum der zugehörige Austrittsort entlang der Längsrichtung des ausgeblendeten streifenförmigen Bereichs zugeordnet wird, und dass die Spektren multivariaten statistischen Analysen unterzogen werden, um die jeweiligen Fettgehalte aus den Spektren in Zuordnung zur Entfernung zwischen Eintritts- und Austrittsorten und damit als Funktion der Tiefe der beteiligten Schichten unter der Haut zu bestimmen.

11. Verfahren nach Anspruch 10, **dadurch gekennzeichnet, dass** die Datenverarbeitungseinheit (10) Spektren von reinem Körperfett, Wasser und Proteinen gespeichert enthält und weiter dazu vorbereitet ist, nach einem Verfahren der multivariaten Statistik die erfassten Spektren aus den Anteilen der gespeicherten Einzelspektren der genannten Substanzen zusammenzusetzen und daraus den relativen Gehalt an Körperfett zu stimmen.

12. Verfahren nach einem der Ansprüche 10 oder 11, **dadurch gekennzeichnet, dass** die Datenverarbeitungseinheit (10) dazu vorbereitet ist, die Längsausdehnung der Blende in eine Mehrzahl von Intervallen zu unterteilen und die Spektren in jedem Intervall zusammengefasst zur Bestimmung des Fettgehalts zu analysieren, jedem Intervall eine Entfernung vom Einstrahlungsort zuzuordnen und die Fettgehalte in den einzelnen Intervallen nach einem vorgegebenem Modell in Fettgehalte als Funktion der Tiefe der beteiligten Gewebeschichten unter der Haut umzurechnen.

## Claims

1. Device for determining the fat content of the human body comprising a light source (3) for radiating light, which light includes a portion in the NIR spectral range, into a body part, a light sensor means (7) for recording light which leaves the body part again at a distance from the radiation position on the same side of the body part, a spectrometer device for differentiated recording of at least two spectral ranges of the light leaving again, and a data processing unit (10), which receives the measuring signals of the light sensor means and processes the signals for determining the fat content of the body part, **characterized in that** the spectrometer device comprises a slot-shaped aperture (5) for masking a slot-shaped portion of the light leaving the body part again at a distance from the radiation position, wherein the aperture is disposed with respect to the light source such that the distance to the radiation position is varying starting from the end of the aperture facing the radiation position along the aperture, and **in that** it comprises a grating (6) configured and disposed such that the dispersive expansion of the light passing through the aperture (5) takes place in a direction different from the longitudinal direction of the aperture, that the light sensor means (7) is configured and disposed in the form of a camera sensor such that the sensor records a diffraction pattern of the grating (6) with the longitudinal direction of the aperture in a first direction and with the dispersive spectral expansion of the light in a second direction different from the first direction on the camera sensor surface, and that the data processing means is further arranged to receive the signals received from the camera sensor (7) as a plurality of spectra corresponding to a plurality of picture elements along the longitudinal direction of the aperture and to subject the plurality of spectra associated with its respective position along the longitudinal extension of the aperture, from which position the respective spectrum origins, to multivariate statistical analysis to determine the respective fat contents from the spectra associated with the distance between the radiation position and the leaving position, which distance light of the respective spectrum has covered, and thereby as a function of depth of the involved layers under the skin.

2. Device according to claim 1, **characterized in that** the grating (6) of the spectrometer is a blazed grating which is optimized such that it delivers dispersed light in the wavelength range from 700 to 1.000 nm concentrated in a predetermined angular range, and that the blazed grating is a reflection or transmission grating formed with an asymmetric sawtooth-shaped surface or a holographic grating.

3. Device according to claim 2, **characterized in that** the blazed grating is a VPH-grating (volume phase holographic grating).

4. Device according any of the preceding claims, **characterized in that** the data processing unit (10) has spectra of pure body fat, water and proteins stored therein, and is further arranged to compose the recorded spectra out of fractions of the stored individual spectra of the substances mentioned, to determine the relative content of body fat based thereon.

5. Device according to any of the preceding claims, **characterized in that** the light source (3) and the aperture (5) are disposed to each other such that the light source radiates light near one end of the slot-shaped aperture, such that the distance from the radiation position to a point along the length of the aperture increases linearly starting from a position adjacent to the light source.

6. Device according to any of the preceding claims, **characterized in that** the data processing unit (10) is arranged to divide the longitudinal extension of the aperture into a plurality of intervals and to analyze the spectra in each interval jointly in order to determine the fat content, to associate each interval with a distance from the radiation position and to convert the fat contents in the individual intervals according to a predetermined model to fat contents as a function of the depth of the tissue layers involved.

7. Device according to claim 6, **characterized in that** the data processing unit (10) is pre-arranged such that each interval, having a distance Xᵢ from the radiation position, is associated with an average depth Tᵢ of the involved tissue layers under the skin according to Tᵢ = Xᵢ/2.

8. Device according to any of the preceding claims, **characterized in that** it comprises a first transparent supporting body (2') having an essentially planar surface for abutting against the skin, through which body light from the light source (3) may be irradiated into the body part to be examined, and a second transparent supporting body (2) having an essentially planar surface for abutting against the skin, through which second transparent body light leaving the body part again impinges on the aperture (5), wherein the two transparent supporting bodies (2, 2') are optically separated from each other such that no direct light can get from the first supporting body (2') into the second supporting body (2).

9. Device according to claim 8, **characterized in that** the planar abutting surfaces of the first (2') and the second transparent supporting body (2) are disposed essentially in a single plane.

10. Method for determining the fat content of a body part of a human body, comprising radiating light that includes a fraction in the NIR spectral range from a light source (3) into the body part (1), recording light leaving the body part again on the same side as the radiation position at a distance from the radiation position by means of a light sensor means (7), wherein a spectrometer unit for differentiated recording of at least two spectral ranges of the light leaving again is provided, and receiving the measuring signals of the light sensor means (7) by a data processing unit (10) and by processing the data for determining the fat content of the body part, **characterized in that**
from the light that is leaving the body part (1) again at a distance from the radiation position a stripe-shaped area is masked in such a manner that the distance from the radiation position to the position of leaving again is increasing along the stripe-shaped area from the end close to the radiation position along the length of the stripe-shaped area,
light passing the stripe-shaped area is subjected to dispersion at a grating (6) in such away that the dispersive expansion of the light passing through the stripe-shaped
area takes place in a direction that is different from the longitudinal direction of the stripe-shaped area,
the diffraction pattern of the grating is recorded with the longitudinal direction of the stripe-shaped area in a first direction and of the dispersive spectral expansion of the light in a second direction different from the first direction by a camera sensor (7), and
the recorded diffraction pattern is analyzed as plurality of spectra along the longitudinal direction of the stripe-shaped area, wherein each spectrum is associated with the respective leaving position along the longitudinal direction of the masked stripe-shaped area, and **in that** the spectra are subjected to multivariate statistical analysis in order to determine the respective fat contents from the spectra in association with the distance between radiation position and leaving position and thereby as a function of the depth of the involved layers under the skin.

11. Method according to claim 10, **characterized in that** the data processing unit (10) contains spectra of pure body fat, water and proteins stored therein and is further arranged to compose the recorded spectra out of fractions of the stored individual spectra of the substances measured, and to determine the relative content of body fat based thereon.

12. Method according to any of the claims 10 or 11, **characterized in that** the data processing unit (10) is arranged to subdivide the longitudinal extension of the aperture into a plurality of intervals and to analyze the spectra in each respective interval jointly for determining the fat content, wherein each interval is associated with a distance from the radiation position, and to convert the fat contents in the respective intervals according to a given model into fat content as a function of depth of the involved issue layers under the skin.

## Revendications

1. Dispositif de détermination de la teneur en graisse du corps humain avec une source de lumière (3) pour irradier de la lumière, dont une fraction se situe dans la plage spectrale du proche infrarouge, jusque dans une partie du corps, avec un dispositif capteur de lumière (7) pour recevoir la lumière ressortant de la partie du corps à distance du point d'irradiation sur le même côté de la partie du corps, avec un dispositif spectrométrique pour la saisie différenciée d'au moins deux plages spectrales de la lumière ressortant et avec une unité de traitement des données (10), qui reçoit des signaux de mesure du dispositif capteur de lumière et les traite pour déterminer la teneur en graisse de la partie du corps, **caractérisé en ce que** le dispositif spectrométrique présente un obturateur (5) en forme de fente pour masquer une zone en forme de fente de la lumière ressortant du point du corps à distance du point d'irradiation, dans lequel l'obturateur est agencé par rapport à la source de lumière de telle sorte que la distance au point d'irradiation varie à partir de l'extrémité tournée vers le point d'irradiation le long de l'obturateur, et un réseau (6) qui est conçu et agencé de sorte que la dispersion de la lumière traversant l'obturateur (5) s'effectue dans une direction différente de la direction longitudinale de l'obturateur, que le dispositif capteur de lumière (7) est conçu et agencé sous la forme d'un capteur de caméra de sorte qu'il capte une image de diffraction du réseau (6) avec la direction longitudinale de l'obturateur dans une première direction et avec la dispersion spectrale de la lumière dans une deuxième direction, différente de la première, sur sa surface de capteur de caméra, et que l'unité de traitement de données est également préparée pour recevoir les signaux reçus par le capteur de caméra (7) sous la forme d'une pluralité de spectres correspondant à une pluralité d'éléments d'image le long de la direction longitudinale de l'obturateur et pour soumettre la pluralité de spectres attribuée au point le long de l'extension longitudinale de l'obturateur, dont provient le spectre respectif, à des analyses statistiques multivariées pour déterminer les teneurs en graisse respectives à partir des spectres attribués à la distance entre les points d'entrée et de sortie, que la lumière du spectre respectif a parcourue, et ainsi en tant que fonction de la profondeur des couches concernées sous la peau.

2. Dispositif selon la revendication 1, **caractérisé en ce que** le réseau (6) du spectromètre est un réseau échelette, qui est optimisé pour délivrer la lumière dispersée dans la plage de longueurs d'onde de 700 à 1 000 nm jusque dans une plage angulaire prédéfinie, et ce de manière concentrée, **en ce que** le réseau échelette est un réseau de réflexion ou de transmission avec une surface conçue de manière asymétrique en dents de scie ou est un réseau holographique.

3. Dispositif selon la revendication 2, **caractérisé en ce que** le réseau échelette est un réseau VPH (volume phase holographic grating).

4. Dispositif selon l'une quelconque des revendications précédentes, **caractérisé en ce que** l'unité de traitement de données (10) contient en mémoire des spectres de graisse corporelle pure, d'eau et de protéines et est en outre préparée pour agencer, selon un procédé de la statistique multivariée, les spectres saisis à partir des fractions des spectres individuels mémorisés des substances mentionnées et pour en déduire la teneur relative en graisse corporelle.

5. Dispositif selon l'une quelconque des revendications précédentes, **caractérisé en ce que** la source de lumière (3) et l'obturateur (5) sont agencés l'un par rapport à l'autre de telle sorte que la source de lumière émet de la lumière près d'une extrémité de l'obturateur en forme de fente, de sorte que la distance du point d'irradiation à un point situé sur la longueur de l'obturateur augmente de manière linéaire à partir de l'extrémité située au voisinage de la source de lumière.

6. Dispositif selon l'une quelconque des revendications précédentes, **caractérisé en ce que** l'unité de traitement de données (10) est préparée pour subdiviser l'extension longitudinale de l'obturateur en une pluralité d'intervalles et analyser les spectres réunis dans chaque intervalle pour déterminer la teneur en graisse, affecter à chaque intervalle une distance au point d'irradiation et convertir les teneurs en graisse dans chacun des intervalles, via un modèle prédéfini, en teneurs en graisse en tant que fonction de la profondeur des couches de tissu sous la peau qui sont concernées.

7. Dispositif selon la revendication 6, **caractérisé en ce que** l'unité de traitement de données (10) est préparée pour affecter à chaque intervalle dont la distance au point d'irradiation est Xᵢ la profondeur moyenne Tᵢ des couches de tissu sous la peau qui sont concernées selon la relation Tᵢ = Xᵢ/2.

8. Dispositif selon l'une quelconque des revendications précédentes, **caractérisé en ce qu'**il présente un premier corps d'application (2') transparent avec une surface sensiblement plane pour l'application sur la peau, à travers lequel de la lumière provenant de la source de lumière (3) peut être irradiée dans la partie du corps à étudier, et un second corps d'application (2) transparent avec une surface sensiblement plane pour l'application sur la peau, à travers lequel de la lumière ressortant de la partie du corps tombe sur l'obturateur (5), dans lequel les deux corps d'application (2,2') transparents sont séparés optiquement l'un de l'autre, si bien qu'aucune lumière directe en provenance du premier corps d'application (2') ne peut atteindre le second corps d'application (2).

9. Dispositif selon la revendication 8, **caractérisé en ce que** les surfaces d'application planes du premier corps d'application (2') transparent et du second corps d'application (2) transparent sont agencées sensiblement dans un plan.

10. Procédé de détermination de la teneur en graisse d'une partie d'un corps humain, dans lequel de la lumière, dont une fraction se situe dans la plage spectrale du proche infrarouge, est irradiée d'une source de lumière (3) jusque dans la partie du corps (1), la lumière ressortant sur le même côté de la partie du corps à distance du point d'irradiation est captée par un dispositif capteur de lumière (7), dans lequel un dispositif spectrométrique est prévu pour la saisie différenciée d'au moins deux plages spectrales de la lumière ressortant et dans lequel les signaux de mesure du dispositif capteur de lumière (7) sont reçus par une unité de traitement de données (10) et traités pour déterminer la teneur en graisse de la partie du corps, **caractérisé en ce que**,
de la lumière qui ressort de la partie du corps (1) à distance du point d'irradiation, une zone en forme de bande est masquée de telle sorte que la distance du point d'irradiation au point de sortie le long de la zone en forme de bande augmente à partir de l'extrémité tournée vers le point d'irradiation de la zone en forme de bande et le long de la zone en forme de bande,
la lumière tombant à travers la zone en forme de bande est soumise à une diffraction sur un réseau (6) de telle sorte que la dispersion de la lumière ayant traversé la zone en forme de bande s'effectue dans une direction différente de la direction longitudinale de la zone en forme de bande,
l'image de diffraction du réseau avec la direction longitudinale de la zone en forme de bande est reçue dans une premier direction et celle avec la dispersion spectrale de la lumière est reçue dans une seconde direction, différente de la première, par un capteur de caméra (7), et
l'image de diffraction reçue est exploitée sous la forme d'une pluralité de spectres le long de la direction longitudinale de la zone en forme de bande, dans lequel à chaque spectre est attribué le point de sortie correspondant le long de la direction longitudinale de la zone en forme de bande masquée, et les spectres sont soumis à des analyses statistiques multivariées pour déterminer les teneurs en graisse respectives à partir des spectres attribués à la distance entre les points d'entrée et de sortie et ainsi en tant que fonction de la profondeur des couches concernées sous la peau.

11. Procédé selon la revendication 10, **caractérisé en ce que**
l'unité de traitement de données (10) contient en mémoire des spectres de graisse corporelle pure, d'eau et de protéines et est en outre préparée pour agencer, selon un procédé de la statistique multivariée, les spectres saisis à partir des fractions des spectres individuels mémorisés des substances mentionnées et pour en déduire la teneur relative en graisse corporelle.

12. Dispositif selon l'une quelconque des revendications 10 ou 11, **caractérisé en ce que** l'unité de traitement de données (10) est préparée pour subdiviser l'extension longitudinale de l'obturateur en une pluralité d'intervalles et analyser les spectres réunis dans chaque intervalle pour déterminer la teneur en graisse, affecter à chaque intervalle une distance au point d'irradiation et convertir les teneurs en graisse dans chacun des intervalles, via un modèle prédéfini, en teneurs en graisse en tant que fonction de la profondeur des couches de tissu sous la peau qui sont concernées.
